Europäisches Patentamt

(19) European Patent Office

Office européen des brevets

(11) EP 0 827 741 A2

## (12) EUROPEAN PATENT APPLICATION

(43) Date of publication:
11.03.1998 Bulletin 1998/11

(51) Int. Cl.$^6$: **A61K 9/70**, A61K 31/19

(21) Application number: 97115071.9

(22) Date of filing: 30.08.1997

(84) Designated Contracting States:
AT BE CH DE DK ES FI FR GB GR IE IT LI LU MC NL PT SE
Designated Extension States:
AL LT LV RO SI

(30) Priority: 05.09.1996 KR 9638430

(71) Applicant:
JE IL Pharmaceutical Co., Ltd.
Seocho-ku, Seoul-si (KR)

(72) Inventors:
• Choi, Hoo Kyun
Kwangjusi (KR)
• Cho, Young Ju
Kwangju-si (KR)

(74) Representative:
Weber, Dieter, Dr. et al
Weber, Dieter, Dr.,
Seiffert, Klaus, Dipl.-Phys.,
Lieke, Winfried, Dr.
Postfach 61 45
65051 Wiesbaden (DE)

(54) **Composition for transdermal delivery system containing nonsteroidal anti-inflammatory drug as an active ingredient**

(57)    This invention relates to a composition for transdermal delivery system containing a non-steroidal anti-inflammatory drug such as propionic acid derivatives (ketoprofen, etc.) and more particularly, to an improved composition for transdermal delivery system wherein said non-steroidal anti-inflammatory drug is dispersed in nonpolar polymers designed to continuously maximize thermodynamic activity and further to increase the skin permeability of said active ingredient using fatty acid, fatty acid ester, fatty acid alcohol, propylene glycol, propylene glycol fatty acid ester, oleic acid, olein alcohol, ethylene glycol monoethylether or its mixture as a solubilizing agent or permeation enhancer, if deemed necessary.

EP 0 827 741 A2

## Description

## BACKGROUND OF THE INVENTION

### Field of the Invention

This invention relates to a composition for transdermal delivery system containing a non-steroidal anti-inflammatory drug such as propionic acid derivatives (ketoprofen, etc.) and more particularly, to an improved composition for transdermal delivery system wherein said non-steroidal anti-inflammatory drug is dispersed in nonpolar polymers designed to continuously maximize its thermodynamic activity and further to increase the skin permeability of said composition using fatty acid, fatty acid ester, fatty acid alcohol, propylene glycol, propylene glycol fatty acid ester, oleic acid, olein alcohol, ethylene glycol monoethyle or its mixture as a solubilizing agent or permeation enhancer, if deemed necessary.

### Description of the Related Art

Ketoprofen is a non-steroidal anti-inflammatory drug with analgesic effect used in arthritis, osteoarthritis and musculoskeletal pain. However, many patients suffer from gastro-intestinal disturbances when ketoprofen is administered orally and it is usually given 3 to 4 times a day due to its short biological half-life. In particular, since arthritis patients are recommended to receive a long-term therapy, the incidence of side-effects has been high. In view of these side effects and inconveniences, other suitable routes of administration except for oral route need to be developed.

When the active ingredients are delivered transdermally, gastrointestinal disturbances may be avoided; Further, their active ingredients are continuously released from polymer matrix at a constant rate, the dosing frequency may be reduced. With the aforementioned advantages, transdermal delivery system has been applied in various drugs in addition to non-steroidal anti-inflammatory drugs. In spite of the additional advantage that transdermal delivery system can avoid first pass metabolism in the liver, said system is being applied only to limited number of drugs having relatively potent efficacy due to the fact that the skin as a body defence mechanism has low permeability to chemical compounds.

The currently marketed drugs for transdermal delivery system are scopolamine, nitroglycerin, clonidine, fentanyl, nicotine and estradiol. Said systems can be largely divided into matrix type and reservoir type; The former is that drugs are dispersed polymer matrix or adhesives, while the latter is to control the release of drugs through a rate-controlling membrane.

As mentioned in the foregoing, the greatest demerit of the transdermal delivery system faces is that the permeation rate of active ingredients via skin is very slow.

Thus, for the development of said dosage form, the permeation rate needs to be enhanced. In particular, the maximization of said permeation rate should be of great significance to some non-steroidal anti-inflammatory drugs with high daily dose such as ketoprofen.

The Korean Patent Unexamined Publication No. 92-21137 discloses a transdermal preparation formulated in such a manner that the acrylic resin having different water absorption capacity is employed to manufacture the multilayer structure and a maximum amount of a drug is solubilized into the adhesive layer contacting with the skin. However, the skin permeability of a drug is not improved through the simple increase of its solubility and said Korean Patent fails to describe the method of sustaining the saturated concentration of a drug in the adhesive layer.

In other words, the absorption process of a drug via skin occurs by simple diffusion and the absorption rate of a drug is generally in proportion to the concentration gradient of a drug but more accurately, said absorption rate is in proportion to the chemical potential of a drug by Fick's principle, as shown below.

$$J \propto \frac{d\mu}{dx}$$

J: Amount of a drug absorbed per area/hour
$\mu$ : Chemical potential of a drug
x: Thickness of membrane

Also, the following equation is established between the chemical potential and thermodynamic activity.

$$\mu = \mu^\circ + RT\ln a$$

Wherein,

$\mu^\circ$ : Chemical potential in standard conditions
R: Gas constant
T: Absolute temperature
a: Thermodynamic activity

Further, the following equation is established between the thermodynamic activity and concentration.

$$a = \gamma' C$$

Wherein,

$\gamma'$ is activity coefficient,
C is concentration.

According to the above equation, it is noted that when drug concentration is low, the activity coefficient becomes 1 and a linear relationship is established between the thermodynamic activity and the concentration. However, when said drug concentration reaches a certain level, the linear relationship is not maintained due to reduction of the activity coefficient. In case of a saturated solution, the thermodynamic activity of a drug is maximized and its value is 1. Therefore, to maximize the permeation rate of a drug, saturated concentration should be sustained continuously during the dosing period.

In addition to the simple diffusion principle of a drug permeation, another way of skin permeation of a drug is carrier mechanism by solvents. To this end, the proper selection of solvent which has high skin permeability and solubility of the drug is required.

Japanese Patent Unexamined Publication No. 7-233050 discloses an topical patch composition containing a non-steroidal anti-inflammatory drug and muscle relaxant as active ingredients but it fails to describe the change in efficacy with the physicochemical properties of adhesives in use. Further, Japanese Patent Unexamined Publication No.6-135828 also discloses an topical patch composition containing polyacrylic acid in powder form and water-soluble polymers as a base but one cannot expect a higher skin permeation since said patent does not consider the thermodynamic activity of active ingredients.

WO No. 93/04677 discloses that styrene-isoprene-styrene copolymer is used as a polymer base and an appropriate mixture of L-menthol and rosin ester derivatives may be used as a solubilizing agent or an enhancer for a non-steroidal anti-inflammatory drug. Nonetheless, some side-effects such as skin irritation may be induced by L-menthol and therapeutic efficacy of its composition may vary according to the types of backing layer.

## SUMMARY OF THE INVENTION

To improve the aforementioned disadvantages, the inventors have endeavoured to develop a novel transdermal delivery system designed to enhance the bioavailability of the active ingredient based on theoretical rationale of transdermal permeation and simple diffusion. As a result, it has been found that non-polar polymer based adhesive can improve the percutaneous absorption of the active ingredient by maximizing thermodynamic activity and at the same time, can provide analgesic transdermal delivery system with ideal physical characteristics such as good skin adhesion, adequate internal cohesive force, good stability for the active ingredient.

The object of present invention is to provide an improved composition for transdermal delivery system of non-steroidal anti-inflammatory drugs which comprises non-polar polymer based adhesives. The said composition may also comprises solvents with the solubility of greater than 50 / for active ingredients as a solubilizing agent or permeation enhancer. The said composition may also comprises tackifiers, plasticizers, and antioxidants, if necessary. The active ingredients is dispersed in non-polar polymer based adhesive layer.

The transdermal delivery systems according to the present invention are excellent in the percutaneous absorption of propionic acid derivatives such as ketoprofen, one class of non-steroidal anti-inflammatory drugs, and also in physical characteristics, so that good analgesic and anti-inflammatory effects can be obtained

## BRIEF DESCRIPTION OF THE DRAWINGS

Fig. 1     is a cross sectional view showing one example of mono layer matrix-type composition for transdermal delivery according to this invention,

Fig. 2     is a cross sectional view showing example of multi layer matrix-type composition for transdermal delivery according to this invention,

## DETAILED DESCRIPTION OF THE INVENTION

Propionic acid derivatives including ketoprofen, one class of non-steroidal anti-inflammatory drugs, have high affinity, therefore high solubility, in acrylic polymer based adhesives due to their lipophilic nature and tend to be retained within the acrylic polymers. Therefore, their release rate from the acrylic polymers is slow and the amount of drug delivered through the skin is small. Based on the above mentioned drawbacks, it is inevitably necessary to use permeation enhancer to improve permeation rate of the active ingredient across the skin. However, since said permeation enhancers have some limit in their effectiveness, other types of polymer base need to be employed.

Unlike the conventional methods, therefore, the inventors have conducted the intensive studies to apply various types of polymers and have found that by the use of non-polar polymer base, thermodynamic activity of active ingredient including ketoprofen can be maximized and release rate of the active ingredient from the polymer can also be maximized due to the difference in physicochemical characteristics between the active ingredient and polymer base. As a result skin permeation rate of the active ingredient was unexpectedly increased and much higher bioavailability was obtained.

Examples of propionic acid derivatives usable in the present invention include flubiprofen, loxoprofen, pranoprofen as well as ketoprofen. It is more desirable to use non-polar polymer base without functional group. Examples of non-polar polymer base usable in the present invention include natural rubber, butyl rubber, polyisobutylene, polystyrene, polyisoprene, polybutadiene, polyethylene/butylene, polyethylene/propylene, and their copolymers. In addition to the above mentioned polymers, other polymers with similar solubility constant may also be used.

As mentioned in the foregoing, according to this invention, the use of non-polar polymer significantly increases the bioavailability, in addition, what is more surprising is when a solvent with the solubility of greater than 50 / for the active ingredient and good skin permeability is added to the composition, the bioavailability can further be increased by improving the dissolution rate of the active ingredient and by carrier mechanism by the solvent. Examples of above mentioned solvent usable in the present invention as solubilizer and/or permeation enhancer include one or more selected from the group consisting of fatty acid, fatty acid ester, fatty alcohol, propylene glycol and its fatty acid ester, and ethylene glycol monoethyl ether. In addition, any lipophilic solvents with the solubility of greater than 50 / for the active ingredient may also be used.

To further enhance the bioavailability according to this invention, various permeation enhancers well-known in the art can be added to the composition. Examples of above mentioned permeation enhancers include alkyl sulfoxide, azone and its derivatives, pyrrolidone and its derivatives, non-ionic and ionic surfactants, fatty acid and fatty acid ester, urea and its derivatives, alcohols and glycols.

Plasticizers or tackifiers may need to be used depending on the type of polymer used. Any plasticizers or tackifiers well-known in the art can be used in the present invention, however, plasticizers should have similar solubility constant with the polymer used in the preparation. Examples of above mentioned plasticizers include polybutene, paraffin wax, petrolatum, coal tar distillate, rosin, chlorinated hydrocarbons and esters, and phthalates with long aliphatic side chains. Examples of above mentioned tackifiers include rosin and its derivatives, hydrocarbon resins, aromatic resins, resin mixture, alkylated phenols, terpenes, terpene phenoloics, ester gums, hydrogenated esters, and low molecular weight polyisobytylene.

The mixture consisting of said components formulated according to this invention is applied on the commercially available backing layer or release liner used for the manufacture of transdermal delivery system.

The amount of each component used in this invention is more specifically explained in the following.

The range of concentration of the active ingredient, propionic acid derivative, is 0.1 to 30 % by weight of the total composition excluding release liner and backing layer, preferably 0.5 to 20 % by weight. Concentrations smaller than 0.1 % cannot bring about sufficient clinical effects but even if added at a concentration higher than 30 %, no substantial improvement can be observed in clinical effects.

The range of concentration of the solvent used as solubilizer and permeation enhancer is 0.01 % to 35 % by weight of the total composition excluding release liner and backing layer, preferably 0.1 % to 15% by weight. Concentrations smaller than 0.01 % cannot bring about sufficient enhancing effect but if added at a concentration higher than 35 %, the value of the product will decline due to inadequate adhesive strength.

The range of concentration of the plasticizer must be controlled depending on the characteristics of base polymer and is usually 0.1 to 60 % by weight of the total composition excluding release liner and backing layer, preferably 1 to 40 % by weight. Concentrations smaller than 0.1 % can bring about loss of flexibility but if added at a concentration higher than 60 %, adhesive strength may decline.

Tackifiers may need to be added to provide adequate adhesive strength depending on the polymer base used. The range of concentration of the tackifier is 0.1 to 70 % by weight of the total composition excluding release liner and backing layer, preferably 1 to 60 % by weight. Concentrations smaller than 0.1 % may not bring about sufficient adhesive strength but if added at a concentration higher than 70 %, it becomes harder to remove from the skin and may leave a residue on the skin.

To prevent oxidative decomposition of the base polymer and the active ingredient, well-known commercially available anti-oxidants such as butylated hydroxytoluene, butylated hydroxyanisole, and tocopherol can be added. The range of concentration of the anti-oxidant is 0.001 to 5 % by weight of the total composition excluding release liner and backing layer, preferably 0.01 to 2 % by weight. Concentrations smaller than 0.001 % cannot bring about sufficient anti-oxidant effects but even if added at a concentration higher than 5 %, no substantial improvement can be observed in anti-oxidant effects.

In addition to aforementioned components, non-polar polymer base is used to make the total content 100 % by weight. If the weight percent of the adhesive polymer base is too low, the adhesive strength may decline and the loading capacity of the polymer base to include the active ingredient may also decline. To overcome above mentioned undesirable decline and to achieve the object of this invention, the weight percent of the polymer base must be increased.

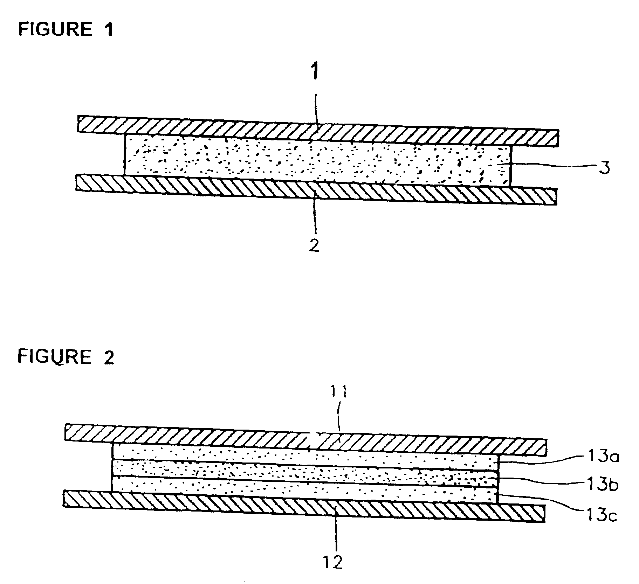

After mixing selected components according to this invention, the mixture can be applied as a mono-layer or as laminated structure of 2 to 5 layer between release liner 1, 11 and backing layer 2, 12 to form substrate layer 3, 13a, 13b, 13c as shown in Figure 1 and Figure 2. In case of laminated multi-layered structure, the active ingredient can be added to single layer or more layers.

The preparation method for the substate layer 3, 13a, 13b, 13c is described as follows: The mixture of selected components to be applied is melted by heat or dissolved in aliphatic hydrocarbon, toluene, chlorinated solvent or their mixture. The said mixture is coated on the release liner 1 at predetermined thickness and transfered to backing layer 2 after proper drying. The said mixture can also be coated directly on the backing layer 2. When multi-layered structure is desired, above mentioned procedure can be repeated. In all cases, the release liner 1, 11 is attached to the opposite side of the backing layer 2, 12. The release liner 1, 11 is removed before the use and applied on the skin. The final product according to this invention is constructed in such a manner that the substrate layer 3, 13a, 13b, 13c of polymer matrix type containing the active ingredient and other additives is enclosed by the release liner 1, 11 and the backing layer 2, 12.

The present invention will be described in detail by the following examples. It should be borne in mind that this invention is by no means limited to or by the examples.

## EXAMPLE 1

Dissolve 3 g of ketoprofen, 28 g of 60 : 40 mixture of low molecular weight(molecular weight range 40,000 ~ 60,000) and medium molecular weigh(molecular weight range 1,000,000 ~ 1,500,000) grade polyisobutylene, 14 g of liquid paraffin, 1.5 g of propylene glycol monolaurate, 28 of butylated hydroxytoluene in hexane/ toluene mixture followed by stirring until complete dissolution. The above mixture was coated on to the release liner to a thickness of 70 μm after drying for 30 minutes at 50 °C (Layer A). In a similar manner to Layer A, dissolve all the above mentioned components except for ketoprofen in hexane. The mixture was coated on to the release liner to a thickness of 70 μm after drying for 30 minutes at 50 °C (Layer B). In a similar manner to Layer B, dissolve all the components of Layer B except for using 50 : 50 mixture of low molecular weight and medium molecular weight grade polyisobutylene. The mixture was coated on to the release liner to a thickness of 70 μm after drying for 30 minutes at 50 °C (Layer C). Layer C was transfered by rolling on to backing layer and removing the release liner afterwards. Subsequently Layer B was laminated on top of Layer C and Layer A on top of Layer B. The release liner attached on Layer A was not removed until the use of the product. The final product containing 30 of ketoprofen was obtained by cutting to the size of 70 cm².

## EXAMPLE 2

In the same manner to example 1 except that propylene glycol monolaurate was replaced by 1.5 g of propylene glycol.

## EXAMPLE 3

In the same manner to example 1 except that propylene glycol monolaurate was replaced by 1.5 g of isopropyl myristate.

## EXAMPLE 4

In the same manner to example 1 except that propylene glycol monolaurate was replaced by 1.5 g of ethylene glycol monoethylether.

## EXAMPLE 5

In the same manner to example 1 except that liquid paraffin was replaced by 14 g of polybutene.

## EXAMPLE 6

In the same manner to example 1 except that Layer C was not used

## TEST

The ketoprofen containing transdermal delivery systems prepared in Examples 1 ~ 6 and a commercial product(Ketoprofen delivery system plaster, KETOTOP) as a control were each applied on the hairless mouse skin to measure the skin permeability. This test used 8 to 12 weeks old hairless mouse and the skin was mounted on the flow-through diffusion cell. The test samples were cut into discs with the surface area of 2 cm² and applied on the skin. The temperature of the receiver cell medium was maintained at 37 °C and pH 7.4 phosphate buffer was used as the receiver cell medium. The samples were collected every 2.5 hours for 20 hours and they were analyzed by High Pressure Liquid Chromatography (HPLC). The composition of the mobile phase was methanol : water : phosphoric acid (800 : 199 : 1). Zorbax Rx C8 was used as the stationary phase. The flux, amount of the drug permeated per unit time and unit area was calculated from the plot of amount permeated versus time. The average fluxes of each Examples for the first 12 hours are shown in Table 1.

Table 1

| Test Samples | Average Flux( /cm²/hr) |
|---|---|
| Example 1 | 17.7 |
| Example 2 | 16.8 |
| Example 3 | 18.0 |
| Example 4 | 15.1 |
| Example 5 | 16.5 |
| Example 6 | 15.8 |
| Commercial Product | 4.7 |

As can be seen in Table 1, the compositions for transdermal delivery system containing a non-steroidal anti-inflammatory drug according to this invention have superior skin permeability to the commercial product since non-polar polymer is being used as the base polymer unlike other conventional product. Further, if necessary, addition of one or more components selected from the group consisting of above mentioned solubilizer, enhancer, plasticizer, and tackifier, may significantly increase the skin permeability of the active ingredient compared to other conventional products.

**Claims**

1. Composition for transdermal delivery system carring thereon non-steroidal anti-inflammatory drug as an active ingredient and polymer base characterized in which one or more anti-inflammatory drugs selected from propionic acid derivatives is dispersed in the non-polar adhesive polymer base.

2. Composition for transdermal delivery system according to claim 1, wherein said non-polar adhesive polymer base is non-polar polymer substance which is free of functional group.

3. Composition for transdermal delivery system according to claim 1 or 2, wherein said non-polar adhesive polymer base without any functional group is selected from natural rubber, butyl rubber, polyisobutylene, polystyrene, polyisoprene, polybutadiene, polyethylene/butylene, polyethylene/propylene and their copolymers, and other polymers with similar solubility constant.

4. Composition for transdermal delivery system according to claim 1, wherein the total content of the non-steroidal anti-inflammatory drug ranges from 0.5 to 30 % by weight of the total composition excluding release liner and backing layer.

5. Composition for tansdermal delivery system according to claim 1, wherein said propionic acid derivative is ketoprofen, flubiprofen, loxoprofen or pranoprofen.

6. Composition for transdermal delivery system according to claim 1 or 5, wherein said propionic acid derivative is ketoprofen.

7. Composition for transdermal delivery system according to claim 1, wherein said composition contains additionally one or more additives selected from the group consisting of solubilizer and permeation enhancer, plasticizer, tackifier, and anti-oxidant.

8. Composition for transdermal delivery system according to claim 7, wherein said solubilizer and permeation enhancer is one or more mixtures selected from the group consisting of fatty acid, fatty acid ester, fatty alcohol, propylene glycol and its fatty acid ester, ethylene glycol monoethyl ether, and any lipophilic solvents with the solubility of greater than 50 / for the said active ingredient.

9. Composition for transdermal delivery system according to claim 7 or 8, wherein the content of said solubilizer and permeation enhancer ranges from 0.01 to 35 % by weight of the total composition excluding release liner and backing layer.

10. Composition for transdermal delivery system according to claim 7, wherein said plasticizer is selected from the group consisting of polybutene, paraffin wax, petrolatum, coal tar distillate, rosin, chlorinated hydrocarbons and esters, and phthalates with long aliphatic side chains.

11. Composition for transdermal delivery system according to claim 7 or 10, wherein the content of said plasticizer ranges from 0.01 to 35 % by weight of the total composition excluding release liner and bakcing layer.

12. Composition for transdermal delivery system according to claim 7, wherein said tackifier is selected from the group consisting of rosin and its derivatives, hydrocarbon resins, aromatic resins, resin mixture, alkylated phenols, terpenes, terpene phenoloics, ester gums, hydrogenated esters, and low molecular weight polyisobutylene.

13. Composition for transdermal delivery system according to claim 7 or 12, wherein the content of said tackifier ranges from 0.1 to 70 % by weight of the total composition excluding release liner and bakcing layer.

14. Composition for transdermal delivery system according to claim 7, wherein said anti-oxidant is selected from the group consisting of butylated hydroxytoluene, butylated hydroxyanisole and tocopherol.

15. Composition for transdermal delivery system according to claim 7 or 14, wherein the content of said anti-oxidant ranges from 0.001 to 5 % by weight of the total comlposition excluding release liner and backing layer.

16. Transdermal delivery system, characterized in which the active ingredient according to claim 1 is dispersed in substrate layer in the form of matrix, or one or more additives are added to the substrate layer in addition to the active ingredient according to claim 7, wherein the substrate layer is applied between release liner and backing layer and is applied as monolayer or 2 ~ 5 multiple layers.

17. Transdermal delivery system according to claim 16, wherein the active ingredient is dispersed in any one or more said substrate multilayers.

FIGURE 1

FIGURE 2